# EUROPEAN PATENT APPLICATION

(11) **EP 3 943 924 A1**
(43) Date of publication of application: **26.01.2022**
(21) Application number: 20187114.2
(22) Date of filing: 22.07.2020
(51) Int. Cl.: G01N 27/02, G01N 33/483

(54) **MICROWELL PLATE FOR IMPEDANCE MEASUREMENTS ON CELL CLUSTERS**

(71) Applicant: Technische Universität München, 80333 München (DE)
(72) Inventor: Brischwein, Martin, 81371 München (DE); Hayden, Oliver, 85368 Moosburg (DE)
(74) Representative: Lucke, Andreas

(57) **Abstract**

Disclosed herein is a microwell plate for impedance measurements on a cluster comprising biological cells, a method for manufacturing the microwell plate, and a method for performing an electrical measurement on a cluster comprising biological cells using the microwell plate. The microwell plate comprises a substrate with a first electrode in a first conductive layer embedded in the substrate, a microwell configured to receive a sample fluid containing biological cells, and a second electrode that is exposed to an inner volume of the microwell and arranged above the first electrode. The microwell comprises a recess extending into the substrate from an upper surface of the substrate to at least the first conductive layer. The recess exposes the first electrode at least in part and is configured to receive the cluster comprising biological cells. Furthermore, the recess is tapered such that a width of the recess parallel to the upper surface of the substrate decreases from a plane between the first and second electrodes to the first first conductive layer.

## Description

### FIELD OF THE INVENTION

The present invention is in the field of biomedical research and clinical diagnostics. In particular, the invention relates to a microwell plate for impedance measurements on a cluster comprising biological cells, a method for manufacturing the microwell plate, and a method for performing an electrical measurement using the microwell plate.

### BACKGROUND

Three-dimensional clusters of biological cells constitute a promising model system in biomedical research and clinical diagnostics, for example in cancer research and oncology, see e.g. L.-B. Weiswald et al, Neoplasia. 17, 1-15 (2015*).* Such cell clusters may for example be formed from cells extracted from a sample of a patient, e.g. a biopsy sample of a tumor, and may be used to assess the effect of drugs prior to a treatment of the patient. The three-dimensional cell clusters may form a tissue-like structure comprising various types of cells and may e.g. reach a higher level of cellular differentiation compared to cell cultures of two-dimensional monolayers. At the same time and in contrast to animal testing, cell clusters are suitable for parallelized high-throughput assays.

The cell clusters may be analyzed using a variety of techniques, for example optical techniques such as fluorescence microscopy or phase-contrast imaging. The cell clusters may also be probed by electrical means, for example by electrical impedance measurements and in particular impedance spectroscopy, see e.g. A. Frank et al., IEEE Reviews in Biomedical Engineering 6, 63-76 (2013*).* In recent years, impedance measurements have been demonstrated on individual cell clusters, for example in microfluidic structures as reported in Y.R.F. Schmid et al., ACS Sensors 1, 1028-1035 (2016*)* and V. F. Curto et al., Lab on a Chip 18, 933-943 (2018*)* and in microcavities on a silicon chip, see D. Kloβ et al, Lab on a Chip 8, 879-884 (2008*)* and EP 2354217 B1.

The approaches proposed so far, however, are challenging to scale up for parallelized high-throughput measurements on a large number of clusters and may not allow for a self-organized growth of cell clusters. Furthermore, the precision of the impedance measurements may be limited, especially for small clusters. The clusters may also be subjected to mechanical forces during transport in microfluidic structures, which may damage the clusters, in particular when exposed to toxic substances, e.g. when testing drugs for chemotherapy.

### SUMMARY OF THE INVENTION

The object of the invention is thus to provide means for precise impedance measurements on clusters comprising biological cells with minimal mechanical stress, which moreover are suitable for parallelized high-throughput analyses.

This object is met by a microwell plate for impedance measurements on a cluster comprising biological cells according to claim 1, a method for manufacturing a microwell plate according to claim 7, and a method for performing an electrical measurement using a microwell plate according to claim 10. Embodiments of the present invention are detailed in the dependent claims.

The microwell plate for impedance measurements on a cluster comprising biological cells according to the invention comprises a substrate with a first electrode in a first conductive layer embedded in the substrate. The microwell plate further comprises a microwell configured to receive a sample fluid containing biological cells and a second electrode that is exposed to an inner volume of the microwell and arranged above the first electrode. The microwell comprises a recess that extends into the substrate from an upper surface of the substrate to at least the first conductive layer. The recess exposes the first electrode at least in part and is configured to receive the cluster comprising biological cells. Furthermore, the recess is tapered such that a width of the recess parallel to the upper surface of the substrate decreases from a plane between the first and second electrode to the first conductive layer.

The substrate may comprise a plurality of dielectric layers, for example at least a lower dielectric layer below the first conductive layer and an upper dielectric layer above the first conductive layer. The first conductive layer may for example be a sheet layer of a conductive material, in particular a metal. The first conductive layer may have been structured or patterned to form conductive structures such as the first electrode, e.g. prior to depositing the upper dielectric layer thereon. In some embodiments, the first conductive layer may comprise other conductive structures in addition to the first electrode, e.g. additional electrodes, a contact pad, and/or one or more conductive traces. The substrate may for example be a printed circuit board, in particular a flexible printed circuit board. The flexible printed circuit board may for example comprise one or more polyimide layers. The substrate may further comprise additional layers, in particular dielectric layers, for example one or more cover layers above and/or below the one or more polyimide layers, a semi-rigid reinforcement layer and/or a solder mask. The cover layers and the reinforcement layer may for example comprise or consist of a polymer material such as polyimide, polycarbonate (PC), polyether ether ketone (PEEK) or a combination thereof. Preferably, some or all of the layers in the substrate are formed of biocompatible materials, which are either intrinsically hydrophilic and/or cell-repellent materials or suitable for a corresponding surface treatment, e.g. as detailed below. In some embodiments, the microwell plate may comprise additional members that are bonded or attached to the substrate, for example a well member as detailed below.

The recess extends into the substrate from an upper surface of the substrate, e.g. through the upper dielectric layer. The recess extends to at least the first conductive layer such that the first electrode is exposed at least in part, i.e. at least one surface of the first electrode is exposed to the inner volume of the microwell. Thereby, a fluid contained in the microwell may come in contact with the first electrode. In some embodiments, the recess extends through the first conductive layer, e.g. into a dielectric layer below the first conductive layer. A bottom of the recess may for example be between 10 µm and 100 µm below the first conductive layer. A similar microwell structure is known from WO 2007/138464 A2, which comprises a dielectric substrate in which a cylindrical through-hole and a plurality of sheet-like electrodes are formed for positioning single cells by dielectrophoresis. On the microwell plate according to the present invention, the recess does not extend through the entire substrate, i.e. at least a portion of the substrate remains below the recess, forming the bottom of the recess. A thickness of the substrate below the bottom of the recess may for example be between 100 µm and 500 µm, e.g. to ensure mechanical stability of the substrate.

The microwell may comprise other portions in addition to the recess, for example an upper portion above the recess, e.g. as detailed below. In other embodiments, the microwell may be formed by the recess, i.e. may only consist of the recess. The microwell encloses the inner volume and is configured to receive a sample fluid containing biological cells therein. The inner volume may for example be between 0.1 µl and 1 ml, e.g. between 1 µl and 100 µl.

The second electrode is exposed to the inner volume of the microwell such that a fluid contained in the microwell may come in contact with the second electrode and may e.g. create an electrical connection between the first and second electrodes. The second electrode is arranged above the first electrode. The second electrode may for example be arranged on or in the substrate or another member of the microwell, e.g. as detailed below. The second electrode comprises or consists of a conductive material, in particular a metal. Preferably, the first and second electrodes comprise or consist of the same material. The second electrode may for example be used as a counter electrode for determining an impedance between the first and second electrodes. In other examples, the second electrode may be used as a shielding electrode for determining an impedance between the first electrode and a third electrode, e.g. as detailed below.

The recess is configured to receive the cluster comprising biological cells, i.e. the width and depth of the recess are chosen such that the cluster can be arranged within the recess at least in part, preferably in its entirety. The cluster is a three-dimensional object comprising a plurality of cells. The cluster may for example be a cell cluster formed by a plurality of cells that adhere to each other and e.g. form an ellipsoidal or approximately ellipsoidal object. The cells may form a tightly-joined tissue-like structure or a loosely-joined cell aggregate. In addition, the cluster may comprise other constituents, for example other biological objects such as extracellular macromolecules or non-biological objects such as a support structure that the cells may be attached to or embedded in. The support structure may for example comprise one or more beads, e.g. collagen-coated beads, and/or a three-dimensional mesh structure. Additionally or alternatively, the support structure may comprise a supporting medium such as a hydrogel. The cluster may for example comprise between 10³ and 10⁵ cells and have a diameter between 100 µm and 1000 µm, in one example between 200 µm and 500 µm. To accommodate the cluster, the depth of the recess and the width of the recess at the upper surface of the substrate may for example be between 100 µm and 1500 µm, in one example between 200 µm and 800 µm.

At least a portion of the recess is tapered, wherein a width of the recess parallel to the substrate's upper surface decreases from at least a plane above the first conductive layer that is parallel to the upper surface and lies between the first and second electrodes to at least the first conductive layer. The width of the recess may decrease continuously from the plane above the first conductive layer to the first conductive layer. In other words, the width along at least one direction parallel to the upper surface, preferably along every direction parallel to the upper surface, and thus a cross-sectional area of the recess may be strictly decreasing from above the first conductive layer to the first conductive layer. One or more sidefaces of the recess may e.g. extend at a non-zero angle relative to a normal vector of the substrate's upper surface at least in part. In other embodiments, the width of the recess may not be strictly decreasing, but may e.g. decrease in discrete steps or only along parts of the tapered portion between the plane above the first conductive layer and the first conductive layer, i.e. the tapered portion of the recess may also comprise one or more portions of constant width. The width of the recess at the first conductive layer may e.g. be less than 50%, in some examples less than 25% of the width at the upper surface along at least one direction, preferably along every direction parallel to the substrate's surface. In some examples, the width of the recess decreases, in one example continuously, from a conductive layer in the substrate above the first conductive layer, e.g. from the conductive layer directly above the first conductive layer or from the uppermost conductive layer in the substrate, to the first conductive layer. In one example, the width of the recess decreases by at least 50 µm, preferably by at least 100 µm within a distance of 100 µm from the first conductive layer.

Providing a tapered recess in the microwell may facilitate aligning the cluster in the microwell relative to the first electrode. The cluster may for example come in contact with portions of one or more angled sidefaces of the recess and may thereby self-align to a well-defined position within the recess, e.g. at the center of the recess. This may in particular allow for arranging the cluster in close proximity to the first electrode. A precise alignment of the cluster may be advantageous for performing measurements on the cluster, in particular for impedance measurement to determine an impedance of the cluster. For example, a fraction of a current between the first and second electrodes that flows through the cluster may strongly depend on the position of the cluster, especially for small cluster sizes, and may thus e.g. be increased by a precise positioning of the cluster in the vicinity of the first electrode.

In some embodiments, the width of the recess parallel to the upper surface of the substrate may also decrease in a bottom portion of the recess, e.g. from the first conductive layer to the bottom of the recess. The width of the recess at its bottom may be less than 20%, in some examples less than 10% of the width at the upper surface along at least one direction, preferably along every direction parallel to the substrate's upper surface. In some embodiments, the width of the recess decreases to zero at the bottom along at least one direction, i.e. sidefaces of the recess may intersect or merge at the bottom.

In some examples, the width of the recess parallel to the upper surface of the substrate decreases continuously from above the first conductive layer to the bottom of the recess. In other words, the width along at least one direction parallel to the upper surface, preferably along every direction parallel to the upper surface, and thus a cross-sectional area of the recess may be strictly decreasing from above the first conductive layer to the bottom of the recess. In some examples, the width of the recess parallel to the upper surface of the substrate decreases continuously from the uppermost conductive layer embedded in the substrate to a bottom of the recess. In yet another example, the entire recess may be tapered and the width of the recess may decrease continuously from the upper surface of the substrate to the bottom of the recess. In some embodiments, the recess may have a concave shape, i.e. both principle curvatures on the sidefaces of the recess may be non-negative, that is zero or positive. Accordingly, any two points on sidefaces of the recess may be connected by a straight line that lies entirely within the recess and/or a sideface thereof. In some examples, at least one principle curvature on the sidefaces of the recess may be positive, wherein the other principle curvature is zero or positive. In a preferred embodiment, the recess has a conical shape. In other examples, both principle curvatures of at least a portion of a sideface may be zero, i.e. the respective portion may be flat. In one example, the recess may have a pyramid-like shape with three or more flat, angled sidefaces, wherein the sidefaces may e.g. intersect at a point at the bottom of the recess. Preferably, the recess is rotationally symmetric, e.g. around an axis perpendicular to the substrate's upper surface, wherein a cross section of the recess in a plane containing the symmetry axis may be concave.

In some embodiments, a depth, a width and/or a shape of the recess are chosen such that a spherical cluster with a reference diameter arranged in the recess is supported by opposing portions of one or more sidefaces of the recess and that a bottom surface of the spherical cluster is aligned with the first conductive layer. In other words, the recess may comprise contact points or contact surfaces on opposing sidefaces that are to come in contact with the spherical cluster, wherein the contact surfaces lie on a sphere with the reference diameter to which the first conductive layer is a tangential plane. This may allow for arranging the cluster comprising biological cells, which may e.g. be spherical or approximately spherical, in close proximity to the first electrode. The contact surfaces may also be tangential to the sphere. In one example, the contact surfaces may form a ring extending around the recess. The reference diameter may for example be between 100 µm and 1000 µm, preferably between 200 µm and 500 µm, e.g. 400 µm in one example.

In some embodiments, a sideface of the recess comprises a cell-repellent coating. The sideface of the recess may e.g. comprise one or more dielectric surfaces and/or an exposed surface of the first electrode. The cell-repellent coating may be arranged on the exposed surface of the first electrode and/or on at least some of the one or more dielectric surfaces. Preferably, all surfaces within the recess are coated with a cell-repellent coating, e.g. all sidefaces of the recess including all exposed surfaces of electrodes in the recess. The cell-repellent coating is configured to reduce an adhesion of cells to the surface that the coating is arranged or deposited on. This may for example be advantageous to facilitate a self-organized formation of clusters within the recess. The cell-repellent coating may for example comprise or consist of polyhydroxyethylmethacrylate, a silicon-based substance such as a silane, and/or polyethylene glycol, e.g. as detailed below. The cell-repellent coating may for example be created by physical and/or chemical surface treatments, e.g. plasma-enhanced chemical vapor deposition. Preferably, the sidefaces of the recess are also hydrophilic and/or comprise a hydrophilic coating, e.g. to prevent the formation of air bubbles within the recess. In some embodiments, the cell-repellent coating maybe hydrophilic.

In some embodiments, the exposed surface of the first electrode, preferably all exposed surfaces of the electrodes in the recess, comprises a biocompatible material. The respective surface may for example comprise a conductive biocompatible coating, e.g. if the first conductive layer comprises a non-biocompatible or toxic material such as copper. The biocompatible coating may comprise or consist of a biocompatible metal or metal alloy, e.g. nickel, palladium and/or a noble metal such as gold. The biocompatible coating or plating may for example be produced with electrodes (e.g. galvanically by electroplating) or without electrodes (e.g. electroless plating) and may for example be arranged below the cell-repellent and/or hydrophilic coatings.

In some embodiments, the substrate comprises more than one conductive layer, e.g. two or three conductive layers. The conductive layers may be electrically isolated from one another and may e.g. be separated by a respective dielectric layer. Each of the conductive layers may comprise conductive structures such as one or more electrodes, conductive traces, and/or contact pads. The conductive layers may be embedded in the substrate. In some examples, one of the conductive layers is arranged on a surface of the substrate, in particular on the upper surface of the substrate.

The substrate may for example comprise a second conductive layer in addition to the first conductive layer, wherein the second conductive layer may e.g. be arranged above the first conductive layer. The second conductive layer may for example be between 10 µm and 100 µm above the first conductive layer, in another example between 400 µm and 800 µm above the first conductive layer. In some embodiments, the second electrode may be formed by the second conductive layer. The recess may for example extend through the second conductive layer and may expose the second electrode at least in part. Preferably, a width of the recess at the second conductive layer is larger than a width of the recess at the first conductive layer. The width of the recess at the second conductive layer may for example be between 10 µm and 200 µm, in another example between 400 µm and 1200 µm larger than the width at the first conductive layer. The width of the recess parallel to the upper surface of the substrate may for example decrease, in some examples continuously, from second conductive layer or from above the second conductive layer to the first conductive layer and/or to the bottom of the recess.

In some examples, the substrate may further comprise a third conductive layer, e.g. above the second conductive layer. The third conductive layer may for example be between 10 µm and 100 µm above the second conductive layer, in another example between 400 µm and 800 µm above the second conductive layer. In one example, the third conductive layer may be arranged on the upper surface of the substrate. The recess may extend through the third conductive layer and may expose a third electrode in the third conductive layer at least in part. Preferably, a width of the recess at the third conductive layer is larger than a width of the recess at the second conductive layer. The width of the recess at the third conductive layer may for example be between 10 µm and 200 µm, in another example between 400 µm and 1200 µm larger than the width at the second conductive layer. The width of the recess parallel to the upper surface of the substrate may for example decrease, in some examples continuously, from third conductive layer or from above the third conductive layer to the first conductive layer and/or to the bottom of the recess. In other embodiments, the third electrode may not be part of the substrate, but may e.g. be arranged in a well member or a cover member.

In some embodiments, the microwell plate further comprises a contact pad that is electrically coupled to the first electrode. The contact pad may be exposed to the surroundings of the microwell plate to provide an electrical connection to the first electrode from the outside. Preferably, the contact pad is arranged on a backside of the substrate opposing the upper surface. In some examples, the contact pad is arranged in and/or on a conductive layer embedded in the substrate, in particular in or on the first conductive layer. The backside of the substrate may comprise a rear-side recess that extends from the backside into the substrate and exposes the contact pad. The contact pad may comprise a conductive pad layer, which may e.g. be deposited on the first conductive layer in the rear-side recess. The microwell plate may comprise further contact pads, e.g. for the second and/or the third electrode, wherein these additional contact pads may be similar to the contact pad for the first electrode.

In some embodiments, the microwell comprises an upper portion above the recess. The upper portion of the microwell has at least one sidewall, wherein the at least one sidewall of the upper portion may extend perpendicular or substantially perpendicular to the upper surface of the substrate, i.e. the width and/or the cross-sectional area of the upper portion may be constant or approximately constant. The upper portion may be arranged in the substrate and/or in another member of the microwell plate. The microwell plate may for example comprise a well member that is arranged on the upper surface of the substrate, e.g. attached or bonded to the upper surface. The well member may comprise an opening or through-hole that is aligned with the recess in the substrate, wherein the opening forms the upper portion of the microwell. A width of the upper portion may be equal to the width of the recess at the upper surface such that the sidewalls of the upper portion are aligned with the sidewalls of the recess. In other examples, the width of the upper portion may be larger than the width of the recess and the upper surface of the substrate may be exposed by the upper portion at least in part. In some embodiments, the second and/or third electrodes may be arranged in or on the well member, e.g. on a sidewall of the upper portion. The well member may for example comprise or consist of plastic, e.g. a thermoplastic polymer material such as polystyrene and/or polypropylene.

In some embodiments, the microwell plate may also comprise a cover member that is configured to cover the microwell, e.g. to close or seal off the inner volume of the microwell. The cover member may for example be configured to be removably placed on the substrate or on the well member. In some examples, the cover member may comprise the second and/or third electrodes.

In some embodiments, the microwell plate further comprises a heating electrode that is electrically isolated from the inner volume of the microwell and configured to conduct an electrical current to adjust a temperature of the microwell. The heating electrode may be embedded in the substrate, in particular below the bottom of the recess, e.g. in a conductive layer below the bottom of the recess. The microwell plate may comprise two contact pads that are electrically coupled to the heating electrode and may for example be arranged on a backside of the substrate, e.g. as described above.

In a preferred embodiment, the microwell plate comprises a plurality of microwells and a plurality of first electrodes, each of which may be formed as described above. Accordingly, each of the first electrodes may be arranged in the first conductive layer and each of the microwells may comprise a tapered recess that extends into the substrate and exposes the respective first electrode at least in part. In some embodiments, the microwells may have different electrode configurations, for example a different number of electrodes and/or a different arrangement of the electrodes, e.g. in different conductive layers of the substrate. The microwells may be arranged in a one-dimensional or two-dimensional array, e.g. in a periodic two-dimensional array. Preferably, the microwells are arranged in an array corresponding to a standardized microplate or microtiter plate layout, e.g. a microplate with 96, 384 or 1536 sample wells. This may allow for automatic handling of the microwell plate with standard microplate handling robots, e.g. a pipetting robot.

Preferably, the first electrodes are electrically isolated from each other, e.g. through an appropriate structuring of the first conductive layer, such that independent measurements can be conducted in each of the microwells. The microwell plate may comprise a respective contact pad for each of the first electrodes. Each of the microwells may further comprise one or more electrodes in additional conductive layers in the substrate, e.g. as described above. In some embodiments, some or all of the microwells may share a common electrode that is exposed to the inner volume of the respective microwells, e.g. the second or third electrode.

In some embodiments, a subset of the microwells may be in fluid connection with each other. Preferably, the microwell plate comprises one or more macrowells, each of which comprises an upper portion providing a fluid connection between a respective subset of the microwells arranged in a bottom portion of the macrowell. The microwells in a macrowell may for example be separated by walls or barriers that extend to a height below an upper edge of the macrowell. If the fluid level in the macrowell is below the upper portion, there may be no fluid exchange between the different microwells, whereas if the fluid level is raised to a level within the upper portion, i.e. above the barriers, fluid exchange between the microwells may take place, e.g. as detailed below. The bottom and/or upper portion of the macrowell may for example be formed by corresponding openings in the well member. A macrowell may for example contain between 2 and 64 microwells. In some examples, the microwells within a macrowell may further be separated into subgroups, wherein microwells within one subgroup are separated by barriers of a first height, whereas microwells in different subgroups are separated by barriers of a second height that is larger than the first height. A subgroup may for example contain between 2 and 32 microwells.

In some embodiments, the microwell plate further comprises an adapter plate with a plurality of connector pins for providing an electrical connection to the first electrodes. The adapter plate may be configured to be removably connected to the microwell plate such that the connector pins are in electrical contact with contact pads associated with a subset of the first electrodes or with all of the first electrodes. The adapter plate may further comprise connector pins that are to be brought in electrical contact with contact pads associated with the second and third electrodes of the corresponding microwells. The adapter plate may further comprise a connector for coupling the connector pins to an electrical interface or a measurement device, e.g. a multi-channel measurement device configured to determine impedances between the respective first and second or third electrodes.

Preferably, the microwell plate further comprises a translation stage that is configured to move the adapter plate relative to the microwell plate. This may for example allow for sequentially contacting different subsets of the first electrodes, e.g. to perform measurements on a large number of microwells using a measurement device with a smaller number of channels. The adapter plate may for example comprise connector pins for simultaneously contacting the electrodes of between 16 and 512 microwells.

The invention further provides a method for manufacturing a microwell plate for impedance measurements on a cluster comprising biological cells according to any one of the embodiments described above. The method comprises (1) providing a substrate with a first electrode in a first conductive layer embedded in the substrate; and (2) forming a microwell configured to receive a sample fluid containing biological cells on the microwell plate, wherein a second electrode is exposed to an inner volume of the microwell and arranged above the first electrode. Forming the microwell comprises forming a recess in the substrate, the recess being configured to receive the cluster comprising biological cells. The recess extends into the substrate from an upper surface of the substrate to at least the first conductive layer and exposes the first electrode at least in part. Furthermore, the recess is tapered such that a width of the recess parallel to the upper surface of the substrate decreases from a plane between the first and second electrodes to the first conductive layer.

The substrate that is provided may have been formed as described above. In particular, the substrate may comprise one or more conductive layers as well as any number of dielectric layers. The conductive layers may have been structured or patterned to form one or more conductive structures, in particular electrodes. The upper surface of the substrate may initially be a flat surface and may e.g. not comprise any recesses and/or protrusions. In some embodiments, the method may comprise forming the substrate at least in part, e.g. structuring one or more conductive layers therein and/or arranging or depositing one or more conductive or dielectric layers thereon. The method may further comprise forming one or more contact pads as described above, e.g. by forming a rear-side recess in the substrate.

Preferably, the method comprises forming a plurality of microwells on the microwell plate, e.g. an array of microwells as describe above. In some examples, the method may further comprise forming one or more macrowells, e.g. as described above. The recess may be formed to have a shape and dimensions as described above. In some examples, the width of the recess parallel to the upper surface of the substrate may also decrease from the first conductive layer to a bottom of the recess. In some embodiments, the width of the recess parallel to the upper surface of the substrate may decrease continuously from above the first conductive layer to the first conductive layer, in one example to the bottom of the recess.

In a preferred embodiment, the recess is formed by drilling. The recess may in particular be formed using a drill bit with a convex and/or tapered shape, e.g. to form the recess with a concave shape as detailed above. In some examples, the recess may additionally or alternatively be formed by milling, embossing, injection molding, laser ablation, etching, and/or photolithography. Additionally or alternatively, the microwell plate or at least a part thereof, in particular the substrate or one or more layers thereof, may be formed by additive manufacturing, in which the recess may be readily formed. Forming the recess may further comprise polishing a sideface of the recess, e.g. to reduce its roughness and to remove drilled material by mechanical and/or chemical polishing.

In some embodiments, the method further comprises coating at least a part of a sideface of the recess with a cell-repellent coating, e.g. coating an exposed surface of the first electrode and/or one or more dielectric surfaces on the sideface with the cell-repellent coating. Preferably, all surfaces within the recess are coated with the cell-repellent coating, e.g. all sidefaces of the recess including all exposed surfaces of the electrodes in the recess. In one example, a first coating process is performed to deposit a first cell-repellent coating on dielectric surfaces on the sideface of the recess and a second coating process is performed to deposit a second cell-repellent coating on the exposed surfaces of the first electrode and/or on exposed surfaces of other electrodes. In another example, the dielectric surfaces and the electrode surfaces may be coated simultaneously in a single coating process, e.g. for example by physical and/or chemical surface treatments, e.g. plasma-enhanced chemical vapor deposition of one or more substances.

Coating the sideface of the recess may for example comprise treating a dielectric surface on the sideface with pre-polymerized polyhydroxyethylmethacrylate (pHEMA) and/or a silicon-based substance such as a silane (i.e. a binary silicon-hydrogen compound), e.g. SiH₄. The recess may for example be exposed to an aqueous solution containing pHEMA and/or the silicon-based substance to allow for an adsorption of pHEMA and/or the silicon-based substance on dielectric layers, in particular polyimide layers, on the sideface of the recess, e.g. to form a self-assembled monolayer or multilayer of pHEMA and/or the silicon-based substance thereon.

Additionally or alternatively, coating the sideface of the recess may comprise treating a dielectric surface on the sideface with polyethylene glycol (PEG). Coating the sideface may for example comprise an adsorption of a substance containing polyethylene glycol (PEG) on the dielectric surface, preferably a covalent bonding of a PEG-containing substance, e.g. on one or more polyimide layers on the sideface of the recess. In some examples, this may comprise chemically activating the dielectric surface by oxidation or reduction, e.g. by exposing the sideface of the recess to an acidic medium and/or to a basic medium or by performing a plasma treatment, e.g. an oxygen plasma treatment. This may also comprise exposing the sideface to a linking agent such as 1-Ethyl-3-(3-dimethylaminopropyl)carbodiimide (EDC) and N-Hydroxysuccinimide (NHS), e.g. for mediating the covalent bonding of amino-functionalized PEG substances on the chemically activated surface of the dielectric layer. Coating the sideface of the recess may also comprise performing a surface cleaning of the sideface by electropolishing, e.g. using copper and sodium carbonate.

Coating the exposed surface of the first electrode may for example comprise an adsorption, in particular a non-covalent adsorption, of a substance containing polyethylene glycol (PEG) on the exposed surface. Preferably, the PEG-containing substance is or comprises thiol-functionalized polyethylene glycol. For this, the exposed surface of the first electrode, which may in particular comprise a noble metal such as gold, may be exposed to a solution containing thiol-functionalized PEG, e.g. to form a self-assembled monolayer or multilayer of PEG on the exposed surface by physisorption and/or chemisorption. Exposed surfaces of other electrodes in the recess may be coated in the same way.

In some embodiments, the method may further comprise coating an exposed surface of the first electrode, preferably all exposed surfaces of electrodes within the recess, with a conductive biocompatible coating, e.g. as described above. The biocompatible coating may for example be applied prior to the cell-repellent coating. The biocompatible coating may for example be deposited on the respective surfaces by one or more physical and/or chemical deposition techniques such as electroplating or electroless plating.

In some embodiments, forming the microwell further comprises bonding a well member to the upper surface of the substrate, wherein the well member comprises an opening that is aligned with the recess. The well member may for example be glued, soldered, and/or mechanically fastened to the substrate and/or bonded to the substrate by ultrasonic welding. Preferably, a plurality of recesses are formed in the substrate and the well member comprises a plurality of openings, each of which is aligned with a respective one of the recesses, thereby forming a plurality of microwells. The openings in the well member may for example form the upper portions of the microwells, e.g. as described above. In some embodiments, the openings in the well member may form lower and/or upper portions of one or more macrowells as described above. In some examples, the method may also comprise forming the well member, e.g. by injection molding, additive manufacturing, and/or milling.

The invention further provides a method for performing an electrical measurement on a cluster comprising biological cells using a microwell plate according to any one of the embodiments as described above. The method comprises (1) arranging the cluster comprising biological cells in the recess of the microwell; and (2) measuring an electrical signal using the first and second electrodes. The above numbering of the steps is for clarity only and does not imply a certain order of execution. As far as technically feasible, the method may be executed in an arbitrary order and steps thereof may be executed simultaneously at least in part.

Measuring the electrical signal may for example comprise measuring a current through or a voltage at at least one of the electrodes of the microwell. Measuring the electrical signal may further comprise applying a voltage to at least one of the electrodes of the microwell, in particular applying a probe voltage between two electrodes of the microwell, for example between the first and second electrodes or the first and third electrodes, and/or applying a shielding voltage to one or more electrodes of the microwell, e.g. to the second electrode. Measuring the electrical signal may further comprise determining one or more quantities from the electrical signal, in particular an impedance, e.g. a direct-current (DC) impedance and/or an alternating-current (AC) impedance. In some examples, the method may comprise providing a measurement fluid in the sample well prior to measuring the electrical signal.

The cluster comprising biological cells may for example have a diameter between 100 µm and 1000 µm, in one example between 200 µm and 500 µm, and may e.g. contain between 10³ and 10⁵ cells. Preferably, the cluster contains cells of different types, e.g. tumor cells, leukocytes, epithelial cells, endothelial cells, and/or fibroblasts. In some examples, at least some of the cells in the cluster may be stem cells. In addition to the cells, the cluster may also comprise other constituents, for example other biological objects such as extracellular macromolecules or non-biological objects such as a support structure that the cells may be attached to or embedded in, e.g. as detailed above. In some examples, the method may comprise providing the cluster as a pre-formed unit, e.g. in a sample fluid, wherein the sample fluid may e.g. also be the measurement fluid. In other examples, the method may comprise forming the cluster, e.g. within the microwell.

Arranging the cluster comprising biological cells in the recess may comprise arranging the cluster on tapered sidefaces of the recess between the first and second electrodes. The cluster may for example be arranged such that the cluster is in contact with and supported by opposing portions of one or more sidefaces of the recess, e.g. in the tapered portion of the recess between the first conductive layer and the plane between the first and second electrodes, in which the width of the recess decreases as detailed above. In one example, the cluster may be in contact with a ring-shaped contact surface extending around the recess on the tapered sidefaces. The cluster may be arranged such that a geometrical center or a center of mass of the cluster lies between the first and second electrodes. Preferably, at least 75% of a length of the cluster, in some examples at least 90% of the length of the cluster lie between the first and second electrodes. In one example, the entire cluster lies between the first and second electrodes, i.e. does not extend below the first electrode and above the second electrode.

Arranging the cluster comprising biological cells in the recess may for example comprise providing a sample fluid containing biological cells in the microwell and holding the sample fluid in the microwell to allow the cluster comprising biological cells to form by self-organized aggregation of cells in the sample fluid. The sample fluid may for example contain individual biological cells and/or small precursor clusters or aggregates of biological cells. The biological cells may for example have been extracted and/or derived from a sample of a patient, e.g. a biopsy sample. Preferably, the sample fluid contains cells of different types, e.g. tumor cells, leukocytes, epithelial cells, endothelial cells, and/or fibroblasts. In one example, the biological cells may comprise circulating tumor cells (CTCs), e.g. platelet-covered CTCs, which may for example have been obtained from the patient by liquid biopsy. In some examples, some or all of the biological cells in the sample fluid may be stem cells. In some examples, the sample fluid may further comprise a support structure or components thereof, e.g. a three-dimensional mesh structure and/or a plurality of beads. Preferably, the sample fluid has a lower density than the biological cells, e.g. such that the cells sink towards the bottom of the microwell due to gravity. To facilitate formation of the cluster, surfaces within the recess are preferably cell-repellent and hydrophilic, e.g. as described above.

In other examples, arranging the cluster comprising biological cells may comprise providing a sample fluid containing the cluster in the microwell. The cluster may for example have been formed beforehand or as part of the method according to the invention, e.g. in a hydrogel matrix, for example by clonal expansion, in a culture medium comprising stem cells that is selectively exposed to one or more growth factors, or by self-organized aggregation on a microwell culture plate.

In some examples, the method may further comprise exposing the cluster comprising biological cells to one or more test substances, for example a pharmacological substance or drug whose effect on cells in the cluster is to be tested. Preferably, the microwell plate comprises a plurality of microwells and the method is executed on the plurality of microwells in parallel. In a preferred embodiment, clusters in different microwells are exposed to different test substances and/or to different doses of the same test substance. The electrical signal may be measured prior to, during, and/or after exposure to the one or more test substances.

In some embodiments, the microwell plate comprises one or more macrowells, e.g. as described above. Each of the macrowells may for example comprise an upper portion providing a fluid connection between a plurality of microwells arranged in a bottom portion of the macrowell. The method may comprise arranging a respective cluster comprising biological cells in the recess of each of the microwells in a macrowell. In some examples, the clusters may be arranged and/or formed while a fluid level in the macrowell is below the upper portion of the macrowell. Accordingly, there may be no fluid exchange between different microwells in the macrowell, e.g. to avoid interference between cells and/or clusters in different microwells.

In some embodiments, the method may further comprise increasing the fluid level to a level within the upper portion of the macrowell to allow for fluid exchange between the microwells in the macrowell, e.g. after arranging the clusters in the recesses. By allowing for fluid exchange between the microwells, substances may for example be applied to all microwells within the macrowell simultaneously. This may reduce the number of pipetting steps required for performing a given experimental sequence. Furthermore, fluid exchange between the microwells may also facilitate interaction between the clusters in different microwells, e.g. by exchange of signaling molecules. This may for example enable the formation of an organ-like structure in the macrowell (organ-on-a chip).

In some embodiments, the method may further comprise providing a calibration fluid in the microwell and calibrating a baseline impedance between two electrodes in the microwell, e.g. between the first and second electrodes, while the respective electrodes are in contact with the calibration fluid. Calibrating the baseline impedance may for example comprise determining an impedance between one or more pairs of electrodes in the microwell, e.g. between the first and second electrodes and/or between the first and third electrodes. The determined impedance values may be stored and subsequently used for correcting impedance values derived from the measurement of the electrical signal. This may for example be advantageous to account for varying baseline impedances among a plurality of microwells. These may e.g. arise due to variations in the shape and/or depth of the corresponding recesses, which may lead to different sizes and locations of the electrodes within the recesses. Preferably, the calibration fluid has a similar or the same composition as a measurement fluid that is used for measuring the electrical signal. The calibration fluid may for example be similar to the sample fluid, but may not contain any cells and/or any clusters.

In a preferred embodiment, the microwell plate comprises at least a third electrode exposed to the inner volume of the microwell. The method may further comprise applying a shielding voltage between a shielding electrode and a counter electrode, wherein the shielding and counter electrodes are selected from the first, second, and third electrodes. The method may further comprise applying a probe voltage between a working electrode and the counter electrode, wherein the working electrode is also selected from the first, second, and third electrodes and is different from the shielding electrode. Preferably, the shielding and probe voltages are applied simultaneously at least in part. The method may also comprise determining an impedance between the working electrode and the counter electrode. The measured electrical signal may for example be the current through the working electrode.

Preferably, the shielding electrode is arranged between the working electrode and the counter electrode. The working electrode may for example be the first electrode in the first conductive layer in the substrate and the shielding electrode may be the second electrode in a second conductive layer above the first conductive layer. The counter electrode may for example be the third electrode, wherein the third electrode may e.g. be arranged in a third conductive layer above the second conductive layer or in a well member bonded to the upper surface of the substrate. Preferably, the shielding electrode has a larger diameter than the working electrode and surrounds the working electrode. A width of the recess at the second conductive layer may for example be between 10 µm and 200 µm larger than at the first conductive layer. The first conductive layer may for example be between 10 µm and 100 µm below the second conductive layer. Preferably, a distance between the working and shielding electrodes is less than 25%, in one example less than 10% of a distance between the working and counter electrodes. A distance between the working and shielding electrodes may for example be smaller than 100 µm, in one example smaller than 20 µm, whereas a distance between the probe and counter electrodes may e.g. be between 400 µm and 1200 µm.

In some embodiments, the cluster may comprise a plurality of biological cells as well as one or more beads. The cluster may for example comprise between 10³ and 10⁵ cells and between 1 and 10⁵ beads. The one or more beads may be randomly distributed throughout the cluster. In some examples, the one or more beads may form a support structure that the cells are attached to and/or embedded in. The beads may be separated from each other and may e.g. be attached to each other through the cells, which may for example adhere to surfaces of the beads as well as to each other, thereby forming the cluster. In another example, a respective cluster may be attached to each of the cells, e.g. for labelling and/or magnetic sorting of the cells. The beads may for example comprise glass, plastic, metal, or a combination thereof and preferably have an insulating surface. A surface of the beads may be coated, e.g. to facilitate attachment of cells to the beads, wherein the coating may for example comprise collagen. Additionally or alternatively, one or more antibodies may be attached to the surface of the beads. In one example, the beads or a part thereof may be magnetic. The beads may for example be spherical or elliptical. The beads may for example have a diameter between 10 µm and 1000 µm, e.g. for forming a suitable support structure, or may have a diameter between 10 nm and 10 µm, e.g. for labelling of the cells.

### LIST OF FIGURES

In the following, a detailed description of the invention and exemplary embodiments thereof is given with reference to the figures. The figures show schematic illustrations of
Fig. 1a: a microwell plate according to an exemplary embodiment of the invention in a cross-sectional side view;
Fig. 1b: the microwell plate of Fig. 1a in top view in accordance with an exemplary embodiment of the invention;
Fig. 2a: a microwell plate with three embedded electrodes according to an exemplary embodiment of the invention in a cross-sectional side view;
Fig. 2b: a microwell plate with three embedded electrodes in an inverted configuration in accordance with an exemplary embodiment of the invention in a cross-sectional side view;
Fig. 2c: a microwell plate with a plurality of vertically stacked electrode pairs according to an exemplary embodiment of the invention in a cross-sectional side view;
Fig. 2d: a microwell plate with a plurality of single-layer electrode pairs and a heating electrode in accordance with an exemplary embodiment of the invention in a cross-sectional side view;
Fig. 3a: a microwell plate with a macrowell according to an exemplary embodiment of the invention in a cross-sectional side view;
Fig. 3b: the microwell plate of Fig. 3a in top view in accordance with an exemplary embodiment of the invention;
Fig. 4: a microwell plate with an adapter plate and a translation stage in accordance with an exemplary embodiment of the invention;
Fig. 5: a flow diagram of a method for manufacturing a microwell plate according to an exemplary embodiment of the invention; and
Fig. 6: a flow diagram of a method for performing an electrical measurement using a microwell plate in accordance with an exemplary embodiment of the invention.

### DESCRIPTION OF THE PREFERRED EMBODIMENTS

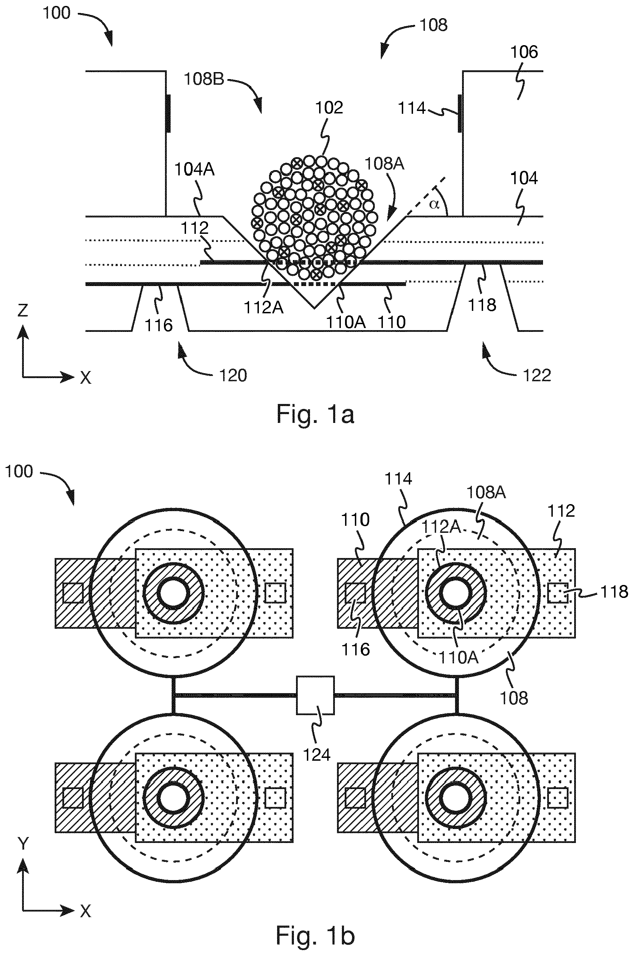

Figs. 1a and 1b depict a schematic illustration of a microwell plate 100 for impedance measurements on a cluster 102 comprising biological cells according to an exemplary embodiment of the invention. Fig. 1a shows the microwell plate 100 in a cross-sectional side view (not to scale) and Fig. 1b in top view (not to scale). The microwell plate 100 may for example have been manufactured using the method 500 described below and may for example be used to perform an electrical measurement on the cluster 102 using the method 600 described below.

The microwell plate 100 comprises a substrate 104 and a well member 106 bonded to a top surface 104A of the substrate 104. The microwell plate 100 further comprises a microwell 108 that is configured to receive a sample fluid containing biological cells, e.g. a sample fluid containing the cluster 102. The microwell 108 comprises a recess 108A extending into the substrate 104 from an upper surface 104A of the substrate 104. The microwell 108 further comprises an upper portion 108B above the recess 108B, which in the example of Fig. 1a is formed by an opening or through-hole in the well member 106 that is aligned with the recess 108A.

The substrate 104 comprises a first electrode 110 in a first conductive layer and a second electrode 112 in a second conductive layer above the first conductive layer. The first and second conductive layers are sheet layers of a conductive material, e.g. copper, and may for example have a thickness between 5 µm and 30 µm. The first and second electrodes 110, 112 have been formed by an appropriate structuring of the first and second conductive layer, respectively. In the example of Fig. 1a, 1b, the first and second electrodes 110, 112 have a rectangular shape and extend from opposite sides of the microwell 108 towards the recess 108A. The first and second electrodes 110, 112 extend below at least a part of the opening formed in the upper surface 104A by the recess 108A, in some examples below the entire opening or the entire upper portion 108B. In some embodiments, one or both of the first and second conductive layers may comprise more than one electrode, e.g. a pair of opposing electrodes arranged adjacent to opposing sidefaces of the recess 108A. Furthermore, the substrate 104 may comprise more than two conductive layers, e.g. as detailed below.

In the example of Fig. 1a, 1b, the substrate 104 is a flexible printed circuit board formed of one or more dielectric materials. The substrate 104 may comprise a plurality of dielectric layers as indicated by the thin dotted lines in Fig. 1a, for example a lower cover layer arranged below the first conductive layer, a polyimide layer arranged between the first and second conductive layers, an upper cover layer arranged above the second conductive layer and a reinforcement layer arranged on top of the upper cover layer or below the lower cover layer. The substrate 104 may for example have a thickness between 500 µm and 2000 µm, wherein the polyimide layer may e.g. have a thickness between 10 µm and 200 µm, the upper and lower cover layers between 50 µm and 300 µm each and the reinforcement layer between 200 µm and 1200 µm. In other examples, the substrate 104 may be a rigid printed circuit board, for example a printed circuit board comprising one or more dielectric layers formed of a composite material such as synthetic resin bonded paper (e.g. FR-2) or glass-reinforced epoxy laminate material (e.g. FR-4).

The recess 108A extends into the substrate 104 from the upper surface 104A through the first and second conductive layers, e.g. into the lower cover layer as illustrated in Fig. 1a. Thereby, the first and second electrodes 110, 112 are exposed at least in part. In the example of Fig. 1a, 1b, the recess 108A has a circular cross-section parallel to the upper surface 104A, i.e. is rotationally symmetric around an axis perpendicular to the upper surface 104A. Accordingly, annular surfaces 110A, 112A of the first and second electrodes 110, 112, respectively, are exposed to the inner volume of the microwell 108 by the recess 108A. In Fig. 1a, portions of the annular surfaces 110A, 112A that do not lie in the sectional plane are shown in broken lines. The sidefaces of the recess 108A including the exposed surfaces 110A, 112A of the first and second electrodes 110, 112 are biocompatible, hydrophilic, and cell-repellent, e.g. by an appropriate choice of the materials in the substrate 104 and/or by providing a biocompatible, hydrophilic, and/or cell-repellent coating thereon, for example as detailed below. Surface areas of the exposed surfaces 110A, 112 may for example be controlled by an appropriate choice of the thickness of the respective conductive layer and/or of the shape of the recess 108A.

The recess 108A is tapered such that a width of the recess 108A and thus a cross-sectional area of the recess 108A parallel to the upper surface 104A decreases from a plane between the first electrode 110 and the second and/or third electrodes 112, 114 to the first conductive layer. The width of the recess 108A may for example decrease continuously from above the first conductive layer to the bottom of the recess 108A as illustrated in Fig. 1a. In the example of Fig. 1a, 1b, the recess has a concave shape, in particular a conical shape, wherein the width of the recess 108A decreases linearly from the upper surface 104A to the bottom of the recess 108A. The sidewalls of the recess 108A may for example extend at an angle α between 30° and 60° to the upper surface 104 A of the substrate 104. Accordingly, a width of the recess 108A at the second conductive layer is larger than a width of the recess 108A at the first conductive layer, for example between 10 µm and 200 µm larger.

The shape and dimensions of the recess 108A are chosen such that the recess is configured to receive the cluster 102 comprising biological cells, i.e. the cluster 102 may be arranged in the recess 108A at least in part as illustrated in Fig. 1a, e.g. such that at least 50%, in some examples at least 90%, in one example all of the cells in the cluster 102 are arranged in the recess 108A. The cluster 102 may for example contain between 10³ and 10⁵ cells, e.g. between 5000 and 10000 cells. In some embodiments, the cluster 102 may further comprise a support structure (not shown) that the cells may be attached to or embedded in, wherein the support structure may for example comprise one or more beads, a three-dimensional mesh structure, and/or a supporting medium such as a hydrogel. The cluster 102 may be approximately spherical and may for example have a diameter between 200 µm and 500 µm. Accordingly, a width of the recess 108A at the upper surface 104A and a depth of the recess 108A from the upper surface 104A may for example be between 200 µm and 800 µm. In the example of Fig. 1a, 1b, the depth of the recess 108A and the slope of the sidefaces of the recess 108A are chosen such that the cluster 102 is supported by opposing portions of the sidefaces of the recess 108A with a bottom surface of the cluster 102 being aligned with the first conductive layer, wherein the diameter of the cluster 102 as shown in Fig. 1a may for example be 400 µm. The thickness of the substrate 104 may for example be chosen such that a remaining portion of the substrate 104 below the bottom of the recess 108A has a thickness between 100 µm and 500 µm.

The microwell plate 100 further comprises contact pads 116, 118 that are electrically coupled to the first and second electrode 110, 112, respectively. The contact pads 116, 118 are arranged on a backside of the substrate 104 opposing the upper surface 104A. In the example of Fig. 1a, the contact pad 116 is arranged in the first conductive layer and the contact pad 118 is arranged in the second conductive layer, wherein the backside of the substrate 104 comprises rear-side recesses 120, 122 exposing a respective one of the contact pads 116, 118. In some embodiments, the contact pads 116, 118 may also comprise a conductive pad layer (not shown). The conductive pad layer may for example be deposited on the respective conductive layer, e.g. within the respective rear-side recess 120, 122, wherein the conductive pad layer may cover sidefaces of the rear-side recess at least in part. In other examples, the conductive pad layer may be deposited on the backside of the substrate 104 and may be electrically connected to the first and second conductive layer, respectively, by a via or interconnect extending from the backside into the substrate 104. The first and second conductive layers may further comprise other conductive structures, for example conductive traces (not shown) connected to the first and second electrode 110, 112, respectively, e.g. to connect the first and second electrodes 110, 112 with the respective contact pad 116, 118.

The well member 106 is bonded to the top surface 104A of the substrate 104, e.g. by providing an adhesive layer (not shown) therebetween. The well member 106 may for example comprise or consist of a thermoplastic polymer material such as polystyrene and/or polypropylene and may for example have a thickness between 1 mm and 10 mm. The well member 106 comprises a cylindrical opening forming the upper portion 108B of the microwell 108, wherein the sidewall of the upper portion 108B extends perpendicular to the upper surface 104A. In the example of Fig. 1a, 1b, a diameter of the upper portion 108B is larger than a diameter of the recess 108A at the upper surface 104A such that the upper portion 108B exposes a part of the upper surface 104A. The diameter of the upper portion 108B may for example be between 1 mm and 5 mm. In other examples, the upper portion 108B may also be tapered and one or more of its sidewalls may be angled relative to a normal vector of the upper surface 104A.

The microwell plate 100 further comprises a third electrode 114, which in the example of Fig. 1a has an annular shape and is arranged on the sidewall of the upper portion 108B such that the third electrode 114 is also exposed to the inner volume of the microwell 108. In other examples, the third electrode 114 may for example be arranged on the top surface 104A of the substrate 104, e.g. on the part of the top surface 104A exposed by the upper portion 108B, or on a backside of the well member 106, e.g. between the substrate 104 and the well member 106. In yet another example, the third electrode 114 may be part of a cover member (not shown) of the microwell plate 100 that is to be placed on the well member 106, e.g. as detailed below. The microwell plate 100 further comprises a third contact pad 124 that is electrically coupled to the third electrode 114. The third contact pad 124 may for example also be arranged on the backside of the substrate 104 and may e.g. be connected to the third electrode 114 through one or more vias extending through the substrate 104. Alternatively, the third contact pad 124 may for example be arranged on a backside of the well member 106 and may e.g. be accessible via a through-hole in the substrate or may be arranged on a top surface of the well member 106 or of the cover member. The electrodes 110-114 may be used for performing an impedance measurement on the cluster 102, for example as detailed below for method 600.

In the example of Fig. 1a, 1b, the microwell plate 100 comprises a plurality of microwells 108 arranged in a two-dimensional array, four of which being shown in Fig. 1b. The microwell plate 100 may for example comprise between 50 and 2000 microwells, wherein the microwells 108 are preferably arranged and shaped so as to match a standardized microplate layout, e.g. a 96-well, 384-well or 1536-well microplate. Each of the microwells 108 has the same structure, namely the one shown in Fig. 1a. In other examples, at least some of the microwells 108 may have different structures, for example different electrode configurations and/or different dimensions of the recess 108A and/or of the upper portion 108B.

The microwell plate 100 comprises a respective contact pad 116, 118 for each of the first and second electrodes 110, 112 in the microwells 108 as illustrated in Fig. 1b. Furthermore, the first and second electrodes 110, 112 are formed in the respective conductive layer such that all of the electrodes 110, 112 are electrically isolated from each other, e.g. such that an independent measurement can be performed for each of the microwells 108. In contrast, the third electrodes 114 of at least some of the microwells 108, in one example of all of the microwells 108 share a common third contact pad 124, e.g. to provide a common voltage reference for each of the microwells 108. In other examples, also the second electrodes 112 of at least some of the microwells 108, in one example of all of the microwells 108 share a common contact pad, e.g. to provide a common shielding voltage for each of the microwells 108.

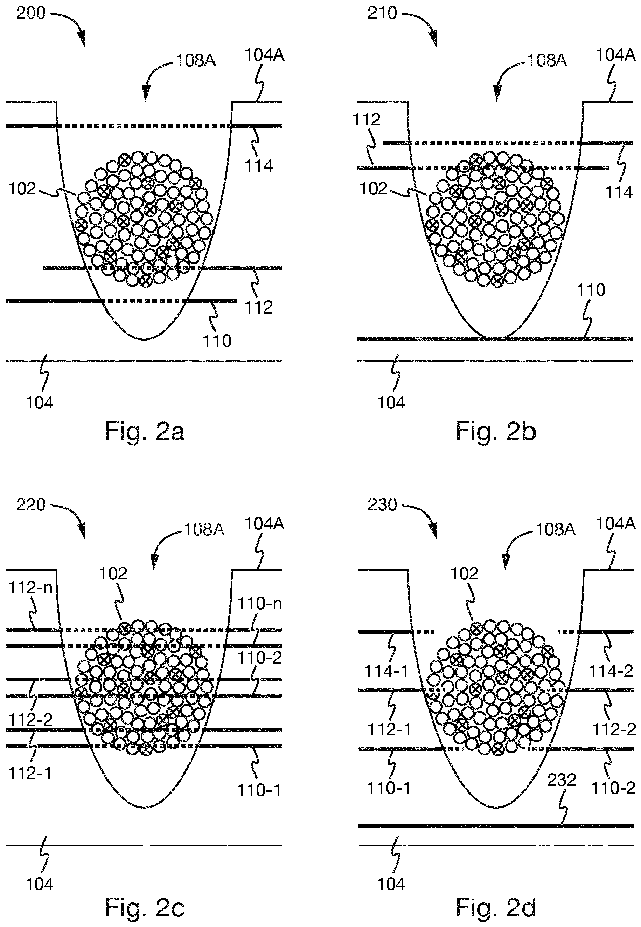

Figs. 2a, 2b, 2c, and 2d depict schematic illustrations of microwell plates 200, 210, 220, and 230, respectively, with different electrode designs according to various embodiments of the invention, wherein the microwell plates 200-230 are shown in a cross-sectional side view (not to scale) as in Fig. 1a.

The substrates 104 of the microwell plates 200-230 are similar to the substrate of the microwell plate 100 of Fig. 1a, 1b. Each of these substrates 104 comprises a number of conductive layers embedded in one or more dielectric layers, e.g. at least one dielectric layer between each pair of adjacent conductive layers. The substrate 104 may be a flexible printed circuit board as in Fig. 1a, 1b or a rigid printed circuit board.

Each of the microwell plates 200-230 comprises a microwell with a tapered recess 108A extending into the substrate 104 from an upper surface 104A thereof. The recesses 108A may for example be rotationally symmetric as in Fig. 1a, 1b. In other examples, the recesses 108A may for example have an elliptical cross-section parallel to the upper surface 104A. The recesses 108A have a concave shape, wherein both principal curvatures on the sidefaces of the recesses 108A are positive. In other words, any two points on the sidefaces may be connected by a straight line that lies entirely within the respective recess 108A. In other examples, the recesses 108A may be conical as in Fig. 1a or may additionally comprise an upper portion with walls that are straight along at least one direction, e.g. a conical upper portion or a cylindrical upper portion. In the examples of Figs. 2a-d, the microwells are formed entirely within the substrate 104, i.e. only comprise the recess 108A and no upper portion as in Fig. 1a. In other examples, the microwell plates 200-230 may also comprise a well member above the substrate 104 similar to the microwell plate 100 of Figs. 1a, 1b. In some embodiments, the microwell plates 200-230 may comprise a plurality of microwells, e.g. similar to the microwell plate 100 of Figs. 1a, 1b.

The microwell plate 200 of Fig. 2a comprises a substrate 104 with three conductive layers, in which a bottom electrode 110, a center electrode 112, and a top electrode 114 are formed, respectively. The bottom and center electrodes 110, 112 are arranged in a bottom portion of the recess 108A and the top electrode 114 is arranged in an upper portion of the recess 108A. In one example, the bottom electrode 110 may be arranged between 10 µm and 100 µm above the bottom of the recess 108A, the center electrode 110 between 10 µm and 100 µm above the bottom electrode, and the top electrode 114 between 400 µm and 800 µm above the center electrode 112. Accordingly, a vertical distance between the bottom and center electrodes 110, 112 may for example be less than 25%, in one example less than 10% of a vertical distance between the center and top electrodes 112, 114. In an impedance measurement, the bottom electrode 110 may for example be used as a working electrode, the center electrode 112 as a shielding electrode, and the top electrode 114 as a counter electrode.

The microwell plate 210 of Fig. 2b also comprises a substrate 104 having three conductive layers with bottom, center, and top electrodes 110, 112, 114. Compared to the microwell plate 200 of Fig. 2a, the configuration is inverted, i.e. the center and top electrodes 112, 114 are both arranged in an upper portion of recess 108B and a vertical distance between the center and top electrodes 112, 114 is smaller than a vertical distance between the bottom and center electrodes 110, 112. In an impedance measurement, the bottom electrode 110 may for example be used as a counter electrode, the center electrode 112 as a shielding electrode, and the top electrode 114 as a working electrode. In the example of Fig. 2b, the recess 108A exposes the bottom electrode 110 in part, but does not extend through the respective conductive layer, i.e. the bottom electrode 110 may form the bottom of the recess 108A.

The microwell plate 220 of Fig. 2c comprises a substrate 104 with a plurality of vertically stacked conductive layers, wherein each conductive layer comprises a respective electrode 110-1, 110-2, ... 110-n, 112-1, 112-2, ... 112-n. The number of conductive layers, 2n, may for example be between 6 and 30. The conductive layers are arranged in pairs, thereby forming pairs of electrodes, each of which comprises a lower electrode 110-i and an upper electrode 112-i with i = 1, 2, ... n. The electrodes 110-1, ... , 112-n may be arranged in a periodic pattern, wherein a vertical distance between the lower and upper electrode of a pair may for example be between 10 µm and 50 µm and a vertical distance between an upper electrode of a first pair and a lower electrode of an adjacent pair above the first pair may for example be between 20 µm and 100 µm. In an impedance measurement, the lower and upper electrodes 110-i, 112-i of each pair may for example be used as a probe and counter electrode, respectively, e.g. to independently determine an impedance between the electrodes for each pair.

The microwell plate 230 of Fig. 2d comprises a substrate 104 having three conductive layers similar to the microwell plates 200 and 210, wherein the three conductive layers are spaced equidistantly in the example of Fig. 2d. In contrast to the microwell plates 100 and 200-220, each of the conductive layers comprises a pair of electrodes 110-1/110-2, 112-1/112-2, 114-1/114-2, wherein the electrodes in each pair are electrically isolated from each other. A first electrode 110-1, 112-1, 114-1 of each pair extends towards the recess 108A from the left and a second electrode 110-2, 112-2, 114-2 of each pair extends towards the recess 108A from the right such that the exposed surfaces of the electrodes in each pair oppose each other. The exposed surface of each of the electrodes 110-1, ... , 114-2 may for example extend along a circular segment around a portion of the recess 108A as indicated in Fig. 2d by the broken lines that partially extend into the recess 108A.

The microwell plate 230 further comprises a heating electrode 232 that is embedded in the substrate 104 and electrically isolated from the recess 108A. The heating electrode 232 may for example be formed in a fourth conductive layer below the bottom of the recess 108A. The heating electrode 232 is configured to conduct an electric current in order to heat the microwell plate 230, in particular the recess 108A. The microwell plate 230 may for example comprise a pair of contact pads (not shown) electrically coupled to the heating electrode 232 for generating the current through the heating electrode 232. The shape of the heating electrode 232 may be adapted to increase an electrical resistance of the heating electrode 232, e.g. to increase the energy dissipated within the heating electrode 232 due to Ohmic losses. In one example, the heating electrode 232 comprises a thin conductive trace, e.g. with a width between 2 µm and 20 µm, wherein the thin conductive trace may form a zig-zag or meandering pattern below the recess 108A. Furthermore, the fourth conductive layer may comprise or consist of a material with a higher electrical resistivity than a material of the other three conductive layers in the substrate 104. The fourth conductive layer may for example comprise or consist of a high-resistance metal or metal alloy such as nichrome (NiCr) or cupronickel (Cu-Ni) and/or a ceramic material such as molybdenum disilicide (MoSi2) or silicon carbide (SiC).

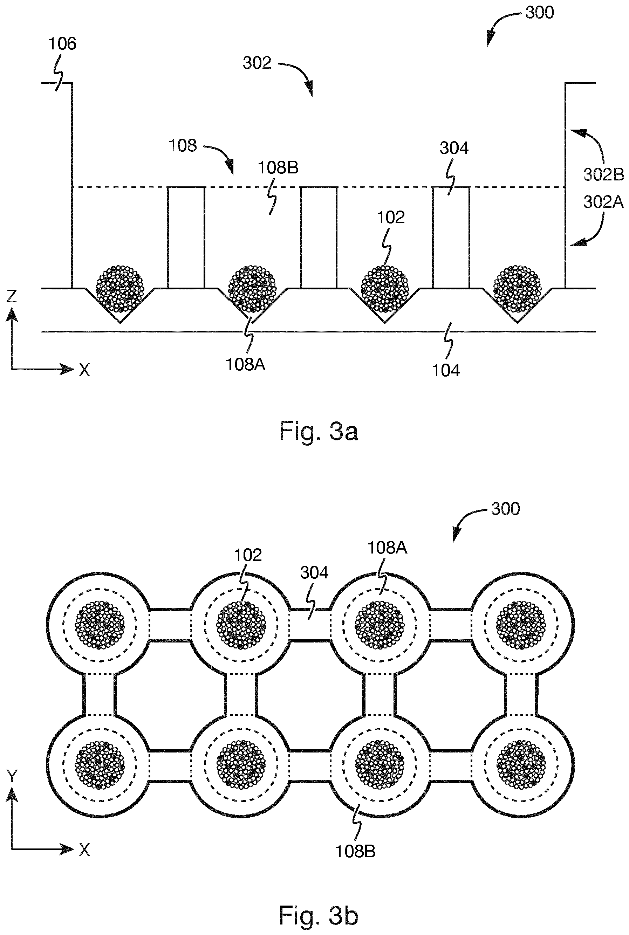

Figs. 3a and 3b depict a schematic illustration of a microwell plate 300 for impedance measurements on clusters 102 comprising biological cells according to an exemplary embodiment of the invention. Fig. 3a shows the microwell plate 300 in a cross-sectional side view (not to scale) and Fig. 3b in top view (not to scale).

The microwell plate 300 comprises a plurality of microwells 108, each of which has a similar structure as the microwells of the microwell plate 100 shown in Fig. 1a. In particular, each of the microwells 108 comprises a tapered recess 108A in a substrate 104 and an upper portion 108B in a well member 106 above the recess 108A. In other examples, at least some of the microwells 108 may be similar to the microwells of one of the microwell plates 200, 210, 220, and 230 described above.

The microwell plate 300 further comprises a macrowell 302, which provides a fluid connection between the microwells 108. The microwells 108 are arranged in a bottom portion 302A of the macrowell 302 and are separated from each other by walls or barriers 304 between adjacent microwells 108. The macrowell 302 further comprises an upper portion 302B above the barriers 304 that provides the fluid connection between microwells 108 in the macrowell 302. The upper portion 302B may for example be a recess in an upper surface of the well member 106, wherein the upper portions 108B of the microwells 108 may be through-holes arranged on a bottom surface of the upper portion 302B. The upper portion 302B may e.g. be a simply connected recess, for example a recess with a rectangular shape with or without rounded corners, or may be a recess forming a network of channels connecting the microwells 108, which may not be simply connected in some examples as illustrated in Fig. 3b.

The barriers 304 may for example extend to a height between 0.5 mm and 5 mm from the upper surface of the substrate 104. The barriers 304 may all have the same height as in the example of Fig. 3a. In other examples, the barriers 304 may have different heights, for example a first subset of the barriers 304 may extend to a first height, e.g. between 0.5 mm and 2 mm, and a second subset of the barriers 304 may extend to a second height that is larger than the first height, e.g. between 2 mm and 5 mm. Thereby, the microwells 108 in the macrowell 302 may be divided into two or more groups, wherein microwells of a given group may be fluidly connected with each other while being separated from microwells in other groups by controlling the fluid level in the macrowell 302, e.g. as detailed below.

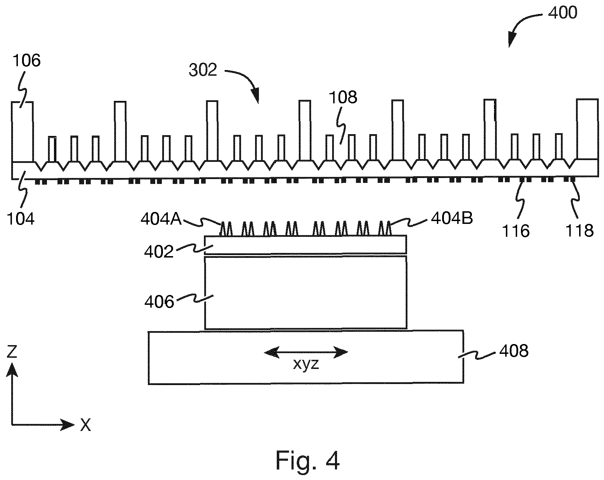

In some embodiments, the microwell plate 300 may comprise a plurality of macrowells, for example between 2 and 64 macrowells, wherein each macrowell may for example comprise between 2 and 64 microwells, e.g. as shown in Fig. 4.

Fig. 4 shows a schematic illustration of a microwell plate 400 in accordance with another exemplary embodiment of the invention in a cross-sectional side view. The microwell plate 400 is similar to the microwell plate 300 and also comprises a substrate 104 and a well member 106. In the well member 106, a plurality of macrowells 302 are formed, each of which comprises a plurality of microwells 108 with a tapered recess extending into the substrate 104. The microwell plate 400 further comprises a plurality of first and second contact pads 116, 118, which may for example be similar to the contact pads shown in Fig. 1a. Each of the first contact pads 116 is electrically connected to a first electrode of a respective one of the microwells 108 and each of the second contact pads 118 is electrically connected to a second electrode of a respective one of the microwells 108.

The microwell plate 400 further comprises an adapter plate 402 with a plurality of first and second connector pins 404A, 404B. The adapter plate 402 is configured to be removably connected to the microwell plate 400 to provide an electrical connection to the electrodes thereon, e.g. to connect an electrical interface 406 to the microwell plate 400. The first and second connector pins 404A, 404B are arranged in pairs, each of which is configured to be electrically connected to the contact pads 116, 118 of one of the microwells 108. When the adapter plate 402 is connected to the microwell plate 400, each of the first connector pins 404A is in electrical contact with a respective one of the first contact pads 116 and each of the second connector pins 404 is in electrical contact with a respective one of the second contact pads 118. In some examples, the connector pins 404A, 404B may comprise elastic elements such as springs, e.g. to facilitate bringing all of the connector pins 404A, 404B into electrical contact with the respective contact pad 116, 118. The adapter plate 402 may further comprise one or more third connector pins (not shown) which are configured to provide an electrical connection to one or more third contact pads on the microwell plate 400, e.g. to connect the electrical interface 406 to a common third electrode of the microwells 108.

The electrical interface 406 may comprise a plurality of channels, each of which may for example be configured to perform an impedance measurement on one of the microwells 108. For this, each channel of the electrical interface may comprise two or more subchannels, e.g. to apply a voltage to the first and/or second electrodes of the respective microwell 108 and/or to measure a current through the first and/or second electrodes. The number of connector pin pairs on the adapter plate 402 may be adapted to the number of channels of the electrical interface 406.

The number of channels of the electrical interface 406 may be smaller than the number of microwells 108 on the microwell plate 400. Accordingly, the electrical interface 406 may only be configured to perform impedance measurements on a subset of the microwells 108. In one example, the number of microwells 108 on the microwell plate 400 is between 512 and 2048 and the number of channels of the electrical interface 406 and thus the number of connector pin pairs on the adapter plate 402 is between 16 and 256. The microwell plate 400 further comprises a translation stage 408 that is configured to move the adapter plate 402 and the electrical interface 406 relative to the microwell plate 400, e.g. to sequentially connect the electrical interface 406 to different subsets of the microwells 108. In the example of Fig. 4, the translation stage 408 is configured to move the adapter plate 402 and the electrical interface 406. In other examples, the translation stage 408 may be configured to move the microwell plate 400 instead or to move the adapter plate 402 only. Preferably, the arrangement of the connector pins 404A, 404B on the adapter plate 402 is adapted to the structure of the macrowells 302 on the microwell plate 400, e.g. such that the adapter plate 402 is configured to provide an electrical connection to all of the microwells 108 in a subset of the macrowells 302.

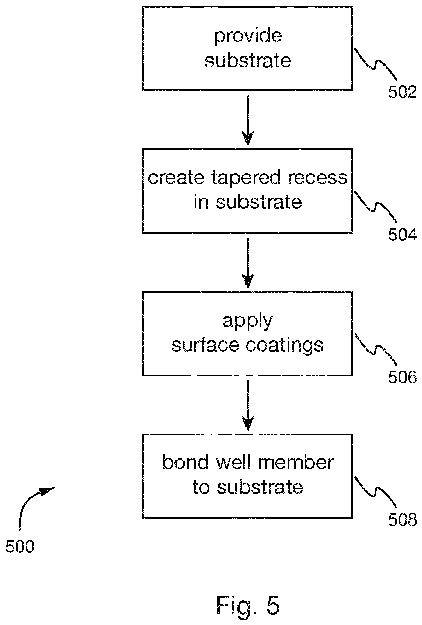

Fig. 5 shows a flow diagram of a method 500 for manufacturing a microwell plate in accordance with an exemplary embodiment of the invention. The method 500 may be used to manufacture a microwell plate for impedance measurements on a cluster comprising biological cells according to any one of the embodiments described herein, for example any one of the microwell plates 100, 200-230, 300, and 400 described above. In the following, the method 500 will be described using the microwell plate 100 of Figs. 1a, 1b as an example for illustration purposes. This is, however, not intended to be limiting in any way. Furthermore, the method 500 is not limited to the order of execution illustrated in the flow diagram in Fig. 5. As far as technically feasible, the method 500 may be executed in an arbitrary order and steps thereof may also be executed simultaneously at least in part.

The method 500 comprises, in step 502, providing a substrate with a first electrode in a first conductive layer embedded in the substrate. For example, the substrate 104 for the microwell plate 100 of Figs. 1a, 1b may be provided, which comprises two electrodes 110, 112 in two conductive layers as described above. The substrate 104 is provided without the recess 108A, e.g. with a flat, unstructured upper surface 104A. In some examples, providing the substrate in step 502 may also comprise forming or structuring the substrate 104 or at least a part thereof, for example forming the rear-side recesses 120, 122, the contact pads 116, 118, and/or the first and/or second electrodes 110, 112, e.g. by patterning the first and/or second conductive layers.

The method 500 further comprises forming a microwell configured to receive a sample fluid containing biological cells on the microwell plate in steps 504, 506, and 508, e.g. forming the microwell 108 on the microwell plate 100 as described in the following. The method 500 may in particular comprise forming a plurality of microwells 108 on the microwell plate 100, e.g. as described above.

In step 504, the tapered recess 108A is formed in the substrate 104, for example by drilling a hole into the upper surface 104A of the substrate 104. Forming the recess 108A by drilling allows for a precise positioning of the recess 108A on the upper surface 104A as well as for a precise control of the depth of the recess 108A. Furthermore, by choosing an appropriate drill bit, e.g. a convex drill bit, in particular a conical drill bit, a variety of rotationally symmetric and/or concave shapes may be realized, for example a conical shape as illustrated in Fig. 1a. Alternatively of additionally, other structuring techniques may be used such as milling, etching, laser ablation, photolithography, and/or additive manufacturing. Step 504 may in particular also comprise polishing sidefaces of the recess 108A, e.g. by chemical and/or mechanical polishing, for example to reduce a surface roughness of the sidefaces.

In the example of Figs. 1a, 1b, the recess 108A extends through the first and second conductive layers and thereby exposes surfaces 110A, 112A of the first and second electrode 110, 112, respectively. The recess 108A further extends through or into at least some of the dielectric layers in the substrate 104. Accordingly, the sidefaces of the recess 108A comprise various conductive and dielectric surfaces. As mentioned above, the sidefaces of the recess 108A should preferably be biocompatible, hydrophilic, and cell-repellent to facilitate the self-organized growth of clusters and a complete wetting of the recess 108A without the formation of air bubbles within the recess 108A. In some embodiments, the materials in the substrate 104 provided in step 502 may have been chosen accordingly. In other examples, a surface treatment may be required to achieve these properties, which may be advantageous as it may increase the choice of materials that can be used in the substrate 104, e.g. to also encompass materials commonly used in printed circuit boards, for example non-biocompatible, toxic metals such as copper and/or dielectrics such as polyimide, which is biocompatible and hydrophilic, but not cell-repellent.

Accordingly, the method 500 may comprise applying one or more surface coatings in the recess 108A in step 506. For example, a conductive biocompatible coating is applied to the exposed surfaces 110A, 112A of the electrodes 110, 112, which may e.g. comprise or consist of copper. One or more biocompatible metals such as nickel, palladium and/or noble metals are deposited on the exposed surfaces 110A, 112A, e.g. by chemical and/or physical deposition techniques. In one example, the exposed surfaces 110A, 112A are first coated with a first coating layer comprising nickel, palladium, and/or gold by electroless plating and subsequently with a second coating layer of gold by electroplating. The first and second coating layer may for example have a thickness between 10 nm and 10 µm each. In a preferred example, the exposed surfaces 110A, 112A are first coated with a first coating layer comprising nickel, e.g. with a thickness between 1 µm and 10 µm, followed by a second coating comprising palladium, e.g. with a thickness between 50 nm and 500 nm, and finally a third coating layer comprising gold, e.g. with a thickness between 20 nm and 100 nm. In one example, step 506 may also comprise passivating an exposed surface of at least one of the electrodes in the microwell 108.

Step 506 may further comprise applying a cell-repellent coating on the exposed surfaces 110A, 112A of the electrodes 110, 112, in particular after applying the conductive biocompatible coating. The cell-repellent coating may for example comprise polyethylene glycol (PEG). A solution containing thiol-functionalized PEG may be provided in the recess 108A. The exposed surfaces 110A, 112A are exposed to this solution for a time that is sufficient to allow the thiol-functionalized PEG to be adsorbed on the exposed surface 110A, 112A, e.g. by non-covalent bonding via sulfur-gold interaction. A coating layer of a desired thickness, e.g. a self-assembled PEG monolayer, may be formed by choosing the exposure time appropriately.

Step 506 may also comprise applying a cell-repellent coating to other portions of the sidefaces of the recess 108A, in particular exposed surfaces of the dielectric layers, for example exposed polyimide surfaces. The cell-repellent coating on the dielectric layers may for example comprise polyhydroxyethylmethacrylate (pHEMA), a silicon-based substance such as a silane or may also comprise PEG. The dielectric surfaces may for example be chemically activated using an acidic or basic medium or by performing a plasma treatment, e.g. an oxygen plasma treatment. Subsequently, the activated surfaces may be chemically modified, e.g. by applying linking agents such as a combination of 1-Ethyl-3-(3-dimethylaminopropyl)carbodiimide (EDC) and N-Hydroxysuccinimide (NHS). This may be followed by a covalent bonding of one or more amino-functionalized substances containing PEG.

The method 500 may further comprise, in step 508, bonding the well member 106 to the substrate 104. The well member 106 may for example comprise a plurality of openings, e.g. as illustrated in Figs. 1, 3, or 4. In some examples, step 508 may comprise forming these openings in the well member 106 or forming the well member 106 itself at least in part, e.g. by injection molding, drilling, milling, and/or additive manufacturing. The well member 106 is arranged on the upper surface 104A of the substrate 104, wherein the openings in the well member 106 are aligned with respective ones of the recesses 108A, e.g. as shown in Figs. 1, 3, or 4. The well member 106 may be bonded to the upper surface 104A by providing an adhesive therebetween. In other examples, the well member 106 may e.g. be welded to the upper surface 104A by ultrasonic welding or may be injection-molded onto the upper surface 104A. In some embodiments, the well member 106 is bonded to the substrate 104 prior to forming the recesses 108A and/or the openings in the well member 106. In one example, the well member 106 may be integrally formed with the substrate 104 and may e.g. be a layer of the substrate 104. In some embodiments, step 508 may also comprise forming an electrical connection between the substrate 104 and the well member 106, e.g. by soldering, for example to connect an electrode in the well member such as the electrode 114 of Figs. 1a, 1b to a contact pad in the substrate, e.g. the contact pad 124.

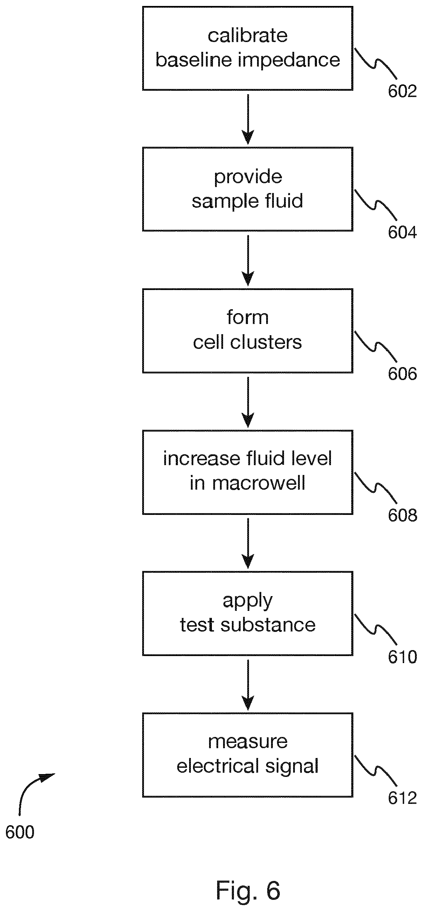

Fig. 6 shows a flow diagram of a method 600 for performing an electrical measurement on a cluster comprising biological cells in accordance with an exemplary embodiment of the invention. The method 600 may be performed using a microwell plate for impedance measurements on a cluster comprising biological cells according to any one of the embodiments described herein, for example any one of the microwell plates 100, 200-230, 300, and 400 described above. In the following, the method 600 will be described using the microwell plates 100 and 300 of Figs. 1a, 1b and 3a, 3b, respectively, as examples for illustration purposes. This is, however, not intended to be limiting in anyway. Furthermore, the method 600 is not limited to the order of execution illustrated in the flow diagram in Fig. 6. As far as technically feasible, the method 600 may be executed in an arbitrary order and steps thereof may also be executed simultaneously at least in part.

The method 600 may in particular be used to perform an impedance measurement on the cluster comprising biological cells, e.g. to determine a direct-current (DC) impedance, an alternating-current (AC) impedance, and/or an impedance spectrum of the cluster. The method 600 may further be executed in parallel for a plurality of clusters, e.g. using a microwell plate with a plurality of microwells. For this, the steps described in the following may be executed on the plurality of microwells simultaneously at least in part.

The method 600 may comprise, in step 602, calibrating a baseline impedance between one or more pairs of electrodes on the microwell plate, wherein the baseline impedance characterizes an impedance of the respective pair of electrodes in absence of the cluster 102. The baseline impedance may in particular be determined between a working electrode and a counter electrode to be used for performing the electrical measurement, for example between the electrodes 110 and 114 in the microwell 108 of Fig. 1a. For this, a calibration fluid is provided in the microwell 108, e.g. an electrolyte solution, in particular an isotonic solution for the cells in the cluster 102. The calibration fluid may for example have the same composition as a sample fluid that the cells are provided in or a measurement solution that is used for determining the electrical signal in step 612. While the electrodes 110 and 114 are in contact with the calibration fluid, the impedance between the electrodes 110, 114 is measured, e.g. by applying a DC or AC voltage between the electrodes 110, 114 and measuring the resulting current through one of the electrodes 110, 114. The calibration may in particular be performed for a variety of frequencies, e.g. at some or all of the frequencies to be used for the measurement of the electrical signal in step 612. The calibration may be performed prior to and/or after executing steps 604 to 612. Performing a calibration of the baseline impedance may for example be advantageous to account for variations in the impedance between the electrodes 110 and 114 due to different depths of the recess 108A, which may lead to different relative positions of the electrodes 110, 112 within the recess 108A as well as to different surface areas of the exposed surfaces 110A, 112A.

The method 600 further comprises arranging a cluster 102 of biological cells in the recess 108A of the microwell 108. For example, a sample fluid containing cells that the cluster 102 is to be formed of is provided in the microwell 108 in step 604, wherein the sample fluid contains the cells as individual cells and/or small precursor clusters or aggregates of cells. The cells may for example have been extracted or derived from a sample of a patient, e.g. from a biopsy sample. The cells may all be of the same type or, preferably, may comprise cells of two or more different types. In some embodiments, the sample fluid may further contain a support structure for the cluster 102 that the cells may attach to or be embedded in, e.g. one or more beads and/or a three-dimensional mesh structure.

Subsequently, in step 606, the cluster 102 may form by self-organized aggregation of cells in the sample fluid in the microwell 108. The cluster 102 may for example grow to a size of between 5000 and 10000 cells, which may e.g. correspond to a diameter of about 300 µm to 400 µm. The density of the sample fluid is chosen to be lower than the density of the cells. Accordingly, the cells may sink downwards in the microwell 108 towards the recess 108A. In this way and due to the tapered sidewalls of the recess 108A, the cluster 102 may self-align with the recess 108A, e.g. at the center of the recess 108A in a plane parallel to the upper surface 104A of the substrate 104, thereby ensuring a well-defined position of the cluster 102 relative to the electrodes 110-114. In particular, no fluid flow may have to be generated to position the cluster 102 relative to the electrodes 110-114, which may reduce the mechanical forces that the cluster 102 is subjected to. In the recess 108A, the cluster 102 may be in contact with and supported by the tapered sidefaces of the recess 108A, e.g. at a point between the first and second electrodes 110, 112 or between the second electrode 112 and the upper surface 104A of the substrate 104. In some examples, the sample fluid may be removed from the microwell 108 after formation of the cluster 102, e.g. to remove cells that are not bound to the cluster 102. In other examples, the sample fluid may remain in the microwell 108 for the measurement of the electrical signal in step 612.

In other embodiments, the cluster 102 may be provided to the microwell 108 as a preformed unit in a sample fluid. The cluster 102 may e.g. have been formed prior to execution of the method 600 or may be formed outside of the microwell 608 as part of the method 600 and subsequently be transferred to the microwell 108. The cluster 102 may e.g. be formed by self-organized aggregation in a microwell culture plate, by clonal expansion in a hydrogel matrix or by selective exposure of stem cells in a culture medium to one or more growth factors. The cluster 102 may directly be placed in the recess 108A, e.g. by targeted pipetting of a microdroplet containing the cluster 102, or may be placed in the microwell 108 and subsequently sink into the recess 108A.

In some embodiments, the microwell 108 may be part of a macrowell, e.g. the macrowell 308 as described above with reference to Figs. 3a, 3b. A respective cluster 102 may be arranged in the recess 108A of some or all of the microwells 108 in the macrowell 302 in steps 604, 606, e.g. as shown in Figs. 3a, 3b. While arranging the cluster 102, the fluid level in the macrowell 308 may be below the upper portion 302B, i.e. below the barriers 304 separating the microwells 108, for example by providing an appropriate amount of sample fluid to the microwells 108. Accordingly, there may be no fluid exchange between different microwells 108 while forming and/or arranging the clusters 102.

The method 600 may further comprise, in step 608, increasing the fluid level in the macrowell 302 to a level within the upper portion 302B, thereby allowing for fluid exchange between the microwells 108 in the macrowell 302. This may for example be advantageous as it facilitates applying the same treatment to each of the clusters 102 within the macrowell 302. All of the clusters 102 in the macrowell 302 may for example be exposed to the same test substance simultaneously in step 610, requiring only a single pipetting step. Furthermore, one can ensure that all clusters 102 within the macrowell 302 are subjected to the same conditions, e.g. the same concentration of the test substance or other substances.

In step 610, one or more test substances are applied to the cluster 102 in the microwell 108. The test substance may for example be a pharmacological substance whose effect on the cells in the cluster 102 is to be assessed, e.g. as a candidate substance for developing a drug in biomedical research or as a potential treatment for a patient in a clinical setting. The test substance may for example be applied by ejecting a droplet containing the test substance into the microwell 108.

In one example, a microwell plate with a plurality of microwells in a plurality of macrowells is used, e.g. the microwell plate 400 of Fig. 4. In each macrowell 302, the clusters 102 may be exposed to the same dose of the same test substance. Different doses of the test substances and/or different test substances may be applied to the different macrowells 302. In this way, a large number of doses and test substances can be tested simultaneously, while at the same time performing multiple instances of the same experiment in each macrowell 302, e.g. to collect statistics and increase the reliability of the experiments.

In step 612, an electrical signal is measured using at least two electrodes in the microwell 108, e.g. the electrodes 110 and 114. The electrical signal may be a voltage between two electrodes or a current through one of the electrodes. For example, a probe voltage is applied between the electrodes 110 and 114 and a current through one of the electrodes is measured to determine an impedance between the electrodes 110 and 114 in the presence of the cluster 102. The current may for example be measured by determining a voltage across a resistor in a supply line to the respective electrode, which may also be referred to as the working electrode. The probe voltage may be a DC voltage or an AC voltage. In some examples, the impedance between the electrodes 110, 114 is determined at a variety of frequencies, e.g. to obtain an impedance spectrum, wherein the frequencies may for example be between 0 Hz and 100 kHz, in one example between 0 Hz and 10 kHz.

Preferably, at least three electrodes are used for performing an impedance measurement, namely a working electrode, a counter electrode and a shielding electrode. The probe voltage is applied between the working and counter electrodes and the current through the working electrode is measured. The shielding electrode is used to direct the current between the working and counter electrodes through the cluster 102. This may for example be advantageous for a small cluster in a medium with an electrical conductivity that is much higher than the conductivity of the cluster since otherwise the current between the working and counter electrodes may predominantly flow through the surrounding medium rather than the cluster itself. To prevent this, a shielding voltage is applied between the shielding electrode and the counter electrode while measuring the current through the working electrode, wherein the shielding voltage may be similar to the probe voltage, e.g. equal to the probe voltage or slightly higher than the probe voltage. For example, the shielding electrode may be connected to the same voltage source as the working electrode, e.g. via a voltage buffer to prevent interference with the current measurement through the working electrode. In some examples, the current through the shielding electrode may not be of interest. To reduce the number of electric contacts or contact pads, the shielding electrodes of a plurality of microwells may thus be connected or short-circuited, e.g. the shielding electrodes of all microwells within a macrowell or of all microwells on the entire microwell plate. The same applies to the counter electrodes. The shielding electrode may be positioned close to the working electrode and preferably has an annular shape and surrounds the working electrode. The shielding electrode and/or the counter electrode may further have a larger surface area than the working electrode in some examples.

Using the microwell plate 100 of Figs. 1a, 1b, this can for example be implemented by using the electrode 110 in the first conductive layer as the working electrode, the electrode 112 in the second conductive layer as the shielding electrode and the electrode 114 as the counter electrode. The same configuration can also be applied to the microwell plate 200 of Fig. 2a, whereas an inverted configuration may be used for the microwell plate 210 of Fig. 2b. In some examples, the counter electrodes of each microwell on the respective microwell plate may be connected with each other, forming a common counter electrode, which may e.g. be connected to ground.

The electrical signal in step 612 is measured after the cluster 102 has been arranged in the recess 108A, i.e. in the presence of the cluster 102. The measurement of the electrical signal may for example be performed after the cluster 102 was exposed to one or more test substances in step 610, e.g. pharmacological substances for the treatment of a medical condition such as cancer. Additionally or alternatively, the measurement may be performed prior to or during exposure to the one or more test substances. The measurement may in particular be repeated multiple times and may also be performed continuously, e.g. to monitor an evolution of the impedance during exposure to the one or more test substances.

In some embodiments, a plurality of electrical signals may be measured between a plurality of electrodes, e.g. using the microwell plate 220 of Fig. 2c. For example, a voltage may be applied between each pair of electrodes 110-i, 112-i with i=i, 2, ..., n and the current through one electrode of each pair may be measured, e.g. through the lower electrode 110-i of each pair. In another example, the method is performed with the microwell plate 230 of Fig. 2d, wherein the center left-hand-side electrode 112-1 is used as a working electrode, the lower and upper left-hand-side electrodes 110-1, 114-1 are used as a shielding electrode and the right-hand-side electrodes 110-2, 112-2, 114-2 are connected and used as a counter electrode.

In some examples, the method 600 may further comprise adjusting a temperature of the microwell 108, e.g. by generating a current through one or more of the electrodes of the microwell plate, for example through the heating electrode 232 of the microwell plate 230 or between two or more electrodes in contact with the microwell 108. The temperature may for example be adjusted to induce a phase transition in a medium in the microwell 108, e.g. in a hydrogel.

The method 600 may also comprise connecting an adapter plate such as the adapter plate 402 of Fig. 4 to the microwell plate 400 to bring the connector pins 404A, 404B into electrical contact with contact pads 116, 118 on the microwell plate 400. The method 600 may in particular comprise bringing the connector pins 404A, 404B into electrical contact with contact pads 116, 118 associated with electrodes of a first subset of microwells 108 on the microwell plate 400, measuring an electrical signal for each of the microwells 108 in the first subset, moving the adapter plate 402 relative to the microwell plate 400, bringing the connector pins 404A, 404B into electrical contact with contact pads 116, 118 associated with electrodes of a second subset of microwells 108 different from the first subset, and measuring an electrical signal for each of the microwells 108 in the second subset.

In addition to impedance measurements, the microwell plates described herein may also be used for other purposes. The microwell plates may for example be used for electrochemical techniques such as chronoamperometry or cyclic voltammetry, e.g. to determine an oxygen concentration in the microwell 108 for assessing a metabolic activity of the cluster 102. In another example, the microwell plates may be used for controlling the motion of cells or clusters by dielectrophoresis.

The embodiments of the present invention disclosed herein only constitute specific examples for illustration purposes. The present invention can be implemented in various ways and with many modifications without altering the underlying basic properties. Therefore, the present invention is only defined by the claims as stated below.

### LIST OF REFERENCE SIGNS

- 100 -: microwell plate
- 102 -: cluster comprising biological cells
- 104 -: substrate
- 104A -: upper surface of the substrate 104
- 106 -: well member
- 108 -: microwell
- 108A -: recess
- 108B -: upper portion of the microwell 108
- 110 -: first electrode
- 110A -: exposed surface of the first electrode 110
- 112 -: second electrode
- 112A -: exposed surface of the second electrode 112
- 114 -: third electrode
- 116 -: first contact pad
- 118 -: second contact pad
- 120, 122 -: rear-side recess
- 124 -: third contact pad

- 200, 210, 220, 230 -: microwell plate
- 232 -: heating electrode

- 300 -: microwell plate
- 302 -: macrowell
- 302A -: bottom portion of the macrowell 302
- 302B -: upper portion of the macrowell 302
- 304 -: barrier

- 400 -: microwell plate
- 402 -: adapter plate
- 404A -: first connector pins
- 404B -: second connector pins
- 406 -: electrical interface
- 408 -: translation stage

- 500 -: method of manufacturing a microwell plate
- 502 -: step of providing a substrate
- 504 -: step of forming a tapered recess
- 506 -: step of applying surface coatings
- 508 -: step of bonding a well member to the substrate

- 600 -: method of performing an electrical measurement using a microwell plate
- 602 -: step of calibrating a baseline impedance
- 604 -: step of providing a sample fluid
- 606 -: step of forming a cluster comprising biological cells
- 608 -: step of increasing the fluid level in a macrowell
- 610 -: step of applying a test substance
- 612 -: step of measuring an electrical signal

## Claims

1. A microwell plate (100) for impedance measurements on a cluster (102) comprising biological cells, the microwell plate (100) comprising:
a substrate (104) with a first electrode (110) in a first conductive layer embedded in the substrate (104);
a microwell (108) configured to receive a sample fluid containing biological cells; and
a second electrode (112, 114) that is exposed to an inner volume of the microwell (108) and arranged above the first electrode (110),
wherein the microwell (108) comprises a recess (108A) extending into the substrate (104) from an upper surface (104A) of the substrate (104) to at least the first conductive layer, wherein the recess (108A):
exposes the first electrode (110) at least in part;
is configured to receive the cluster (102) comprising biological cells; and
is tapered such that a width of the recess (108A) parallel to the upper surface (104A) of the substrate (104) decreases from a plane between the first and second electrodes (110, 112, 114) to the first first conductive layer.

2. The microwell plate (100) of claim 1, wherein:
the width of the recess (108A) parallel to the upper surface (104A) of the substrate (104) decreases from the first conductive layer to a bottom of the recess (108A), in particular wherein the width of the recess (108A) parallel to the upper surface (104A) of the substrate (104) decreases continuously from above the first conductive layer to the bottom of the recess (108A); and/or
the recess (108A) has a concave shape, in particular a conical shape; and/or
a depth, a width and/or a shape of the recess (108A) are chosen such that a spherical cluster (102) with a reference diameter arranged in the recess (108A) is supported by opposing portions of one or more sidefaces of the recess (108A) and a bottom surface of the spherical cluster (102) is aligned with the first conductive layer, the reference diameter being between 100 µm and 1000 µm, preferably between 200 µm and 500 µm.

3. The microwell plate (100) of claim 1 or 2, wherein:
a sideface of the recess (108A) comprises a cell-repellent coating; and/or
the substrate (104) comprises a second conductive layer above the first conductive layer, the second electrode (112) being formed by the second conductive layer, wherein a width of the recess (108A) at the second conductive layer is larger than a width of the recess (108A) at the first conductive layer.

4. The microwell plate (100) of any one of the preceding claims, further comprising a contact pad (116) on a backside of the substrate (104), the contact pad (116) being electrically coupled to the first electrode (110), in particular wherein the contact pad (116) is arranged in and/or on the first conductive layer and the backside of the substrate (104) comprises a rear-side recess (120) exposing the contact pad (116).

5. The microwell plate (100) of any one of the preceding claims, wherein:
the microwell (108) comprises an upper portion (108B) above the recess (108A) having at least one sidewall, wherein the at least one sidewall of the upper portion (108B) extends substantially perpendicular to the upper surface (104A) of the substrate (104); and/or
the microwell plate (230) further comprises a heating electrode (232) embedded in the substrate (104) below the bottom of the recess (108A).

6. The microwell plate (100) of any one of the preceding claims, wherein the microwell plate (100) comprises a plurality of microwells (108) and a plurality of first electrodes (110), the first electrodes (110) being electrically isolated from each other, in particular wherein:
the microwell plate (300) comprises a plurality of macrowells (302), each of which comprises an upper portion (302B) providing a fluid connection between a respective subset of the microwells (108) arranged in a bottom portion (302A) of the macrowell (302); and/or
the microwell plate (400) further comprises an adapter plate (402) with a plurality of connector pins (404A, 404B), the adapter plate (402) being configured to be removably connected to the microwell plate (400) such that the connector pins (404A, 404B) are in electrical contact with contact pads (116) associated with a subset of the first electrodes (110), wherein the microwell plate (400) preferably further comprises a translation stage (408) that is configured to move the adapter plate (402) relative to the microwell plate (400).

7. A method (500) for manufacturing a microwell plate (100) for impedance measurements on a cluster (102) comprising biological cells according to any one of the preceding claims, the method (500) comprising:
providing a substrate (104) with a first electrode (110) in a first conductive layer embedded in the substrate (104); and
forming a microwell (108) configured to receive a sample fluid containing biological cells on the microwell plate (100), wherein a second electrode (112, 114) is exposed to an inner volume of the microwell (108) and arranged above the first electrode (110),
wherein forming the microwell (108) comprises forming a recess (108A) in the substrate (104), the recess (108A) being configured to receive the cluster (102) comprising biological cells, wherein the recess (108A):
extends into the substrate (104) from an upper surface (104A) of the substrate (104) to at least the first conductive layer and exposes the first electrode (110) at least in part; and
is tapered such that a width of the recess (108A) parallel to the upper surface (104A) of the substrate (104) decreases from a plane between the first and second electrodes (110, 112, 114) to the first conductive layer.

8. The method (500) of claim 7, wherein:
the width of the recess (108A) parallel to the upper surface (104A) of the substrate (104) decreases from the first conductive layer to a bottom of the recess (108A), in particular wherein the width of the recess (108A) parallel to the upper surface (104A) of the substrate (104) decreases continuously from above the first conductive layer to the bottom of the recess (108A); and/or
the recess (108A) is formed by drilling.

9. The method (500) of claim 7 or 8, wherein:
the method (500) further comprises coating at least a part of a sideface of the recess (108A) with a cell-repellent coating, in particular wherein coating the sideface of the recess (108A) comprises treating a dielectric surface on the sideface with a silane and/or polyethylene glycol and/or coating an exposed surface (110A) of the first electrode (110) by adsorption of thiol-functionalized polyethylene glycol on the exposed surface; and/or
forming the microwell (108) further comprises bonding a well member (106) to the upper surface (104A) of the substrate (104), wherein the well member (106) comprises an opening (108B) that is aligned with the recess (108A).

10. A method (600) for performing an electrical measurement on a cluster (102) comprising biological cells using a microwell plate (100) according to any one of claims 1 to 6, the method (600) comprising:
arranging the cluster (102) comprising biological cells in the recess (108A) of the microwell (108); and
measuring an electrical signal using the first and second electrodes (110, 112, 114).

11. The method (600) of claim 10, wherein arranging the cluster (102) comprising biological cells in the recess (108A) comprises:
arranging the cluster (102) comprising biological cells on tapered sidefaces of the recess (108A) between the first and second electrodes (110, 112, 114); and/or providing a sample fluid containing biological cells in the microwell (108) and holding the sample fluid in the microwell (108) to allow the cluster (102) comprising biological cells to form by self-organized aggregation of cells in the sample fluid.

12. The method (600) of claim 10 or 11, wherein the microwell plate (300) comprises a plurality of macrowells (302), each of which comprises an upper portion (302B) providing a fluid connection between a plurality of microwells (108) arranged in a bottom portion (302A) of the macrowell (302), the method (600) comprising:
arranging a respective cluster (102) comprising biological cells in the recess (108A) of each of the microwells (108) in a macrowell (302) while a fluid level in the macrowell (302) is below the upper portion (302B) of the macrowell (302) such that there is no fluid exchange between different microwells (108) in the macrowell (302); and
increasing the fluid level to a level within the upper portion (302B) of the macrowell (302) to allow for fluid exchange between the microwells (108) in the macrowell (302).

13. The method (600) of any one of claims 10 to 12, further comprising:
providing a calibration fluid in the microwell (108); and
calibrating a baseline impedance between two electrodes (110, 112, 114) in the microwell (108) while the respective electrodes (110, 112, 114) are in contact with the calibration fluid.

14. The method (600) of any one of claims 10 to 13, wherein the microwell plate (100) comprises a third electrode (114) exposed to the inner volume of the microwell (108), the method (600) further comprising:
applying a shielding voltage between a shielding electrode (112) and a counter electrode (114), the shielding and counter electrodes (112, 114) being selected from the first, second, and third electrodes (110, 112, 114);
applying a probe voltage between a working electrode (110) and the counter electrode (114), the working electrode (110) being selected from the first, second, and third electrodes (110, 112, 114) and being different from the shielding electrode (112); and
determining an impedance between the working electrode (110) and the counter electrode (114).

15. The method (600) of any one of claims 10 to 14, wherein the cluster (102) comprises a plurality of biological cells and one or more beads.
